# EUROPEAN PATENT APPLICATION

(11) **EP 4 344 646 A1**
(43) Date of publication of application: **03.04.2024**
(21) Application number: 22315223.2
(22) Date of filing: 30.09.2022
(51) Int. Cl.: A61B 8/00, A61B 8/08, A61B 5/00

(54) **METHOD AND SYSTEM FOR ADJUSTING A MEDICAL IMAGING PARAMETER OF A MEDIUM**

(71) Applicant: SUPERSONIC IMAGINE, 13290 Aix en Provence (FR)
(72) Inventor: Lavaud, Jonathan, 13090 Aix en Provence (FR); Zhang, Bo, 13109 Simiane-Collongue (FR)
(74) Representative: Paustian & Partner Patentanwälte mbB

(57) **Abstract**

The invention relates to a method (100) of adjusting a medical imaging parameter of a medium, wherein the method (100) comprises:
obtaining (a) an optical characteristic of the medium,
adjusting (b) the medical imaging parameter as a function of the optical characteristic.

## Description

### BACKGROUND

It is known to use transducer for communication, imaging or scanning purposes, for example in the field of medical imaging, radar, sonar, seismology, wireless communications, radio astronomy, acoustics and biomedicine. One example comprises ultrasound imaging.

The aim of ultrasound imaging is to estimate the medium reflectivity. In a conventional ultrasound imaging method, an ultrasound transducer device (also referred to as an ultrasound probe) with a set of ultrasound transducer elements may be used. In the method, one or multiple transducers are used to transmit one or successively several ultrasound beam into a medium, corresponding to a transmission operation. Then, in a reception operation a set of backscattered echo signals are received from the medium by the same set or another set of transducer elements. In particular, each of the transducer elements converts a received echo signal into for example an electrical signal. The signal may further be processed by the ultrasound system or by any associated (optionally dedicated) system. For example, they may be amplified, filtered digitalized and/or a signal conditioning operation may be carried out. The transducer elements may be arranged as a transducer line or as a transducer array or any other configuration.

Conventionally, said signals are then transmitted to an image processing system. The received signals may be processed to generate image data of the scanned medium, for example using a beamforming method, more in particular a delay and sum (DAS) beamforming method. Generally, beamforming may be understood as a signal processing technique conventionally used in sensor arrays for directional signal transmission and/or reception. This process is used to generate beamformed data. In other words: beamforming may be understood as a signal processing technique that is achieved by combining elements in an antenna array (for example an ultrasound transducer) in such a way that the combined signals form constructive interferences.

### SUMMARY OF THE DISCLOSURE

The system and method described herein are related to technologies for adjusting a medical imaging parameter of a medium.

A method of adjusting a medical imaging parameter of a medium is provided. The method comprises obtaining an optical characteristic of the medium. The method also comprises adjusting the medical imaging parameter as a function of the optical characteristic.

The optical characteristic may be obtained, for example, in an optical acquisition method. The optical acquisition may be done by an optical spectrometer or optical element(s), for example an optical sensor. An optical element may comprise borescopes, fiberscopes, fiber optic faceplates, lenses, prisms, mirrors, light-emitting diodes, photodetectors, optical fibers, fiber optic cables, and/or fiber optic bundles.

An optical characteristic may be any type of characteristic that may be received by optical acquisition. For example, it may be possible that the optical characteristic of the medium is obtained by optical acquisition using light of one wavelength. Also different wavelengths may be used.

By adjusting, any type of change, determination and/or calculation may be meant. Any type of mathematical operation may be used for this purpose, in particular also iterative methods. Reading from tables may also be used to adjust the medical imaging parameter. Adjusting the medical imaging parameter may also be carried out using a mathematical model. A mathematical model may also comprise a machine learning model. In other words, the adjustment may be implemented or may use an artificial-intelligence-based (AI-based) algorithm and/or (pre-trained) machine learning algorithm.

The medical imaging parameter and the optical characteristic may be associated both with the same spatial region of the medium. A spatial region of the medium may also be defined as region of interest (ROI). For example, the medical imaging parameter and the optical characteristic may be both determined for the same ROI of a medium. It is also possible that the medical imaging parameter may be determined for a ROI of the medium and the optical characteristic may be determined as an overall characteristic of the medium.

One advantage of such a method is the possibility to improve the quality of the medical image data and in a fast and/or computationally efficient manner. In particular, adjusting the medical imaging parameter with information about the optical characteristic of the medium may increase quality of the medical image data. For example, the optical characteristic may comprise a property of the medium. By knowing the property of the medium, the medical imaging parameter may be adjusted to improve the quality of the medical imaging data.

In general, the obtained optical parameter allows a medical imaging method to become adaptive to particular medium characteristics.

Moreover, a real-time adjustment or quasi-real-time adjustment (i.e. where a user does not perceive and delay due to the adjustment) becomes advantageously possible, for example because the optical characteristic may be obtained in advance or simultaneously with an acquisition of the medical data (for example ultrasound data).

Another advantage is that an additional marker may be determined for medical imaging when taking into account the optical characteristic. An additional marker may help for a more reliable detection of tumours or the like. Additional markers may further help for all kinds of medical imaging methods, for example ultrasound imaging. In particular ShearWave^{™} Elastography (SWE) for determining elasticity may be improved by using the system or method according to any examples of the present disclosure, as described in some examples below.

The method may be used for the examination of any kind of any tissue or organ. For example, the method may be used for breast or liver. Furthermore, the method may be used to examine musculoskeletal regions of the bodies. For example, the method may be used to examine liquid present in certain regions of a body like the knees.

In one aspect, the optical characteristic may comprise at least one of a light absorption characteristic, a light scattering characteristic, and a light diffusion characteristic of the medium.

The optical characteristic may optionally comprise a light absorption characteristic, light scattering characteristic, and/or light diffusion characteristic of the medium as a function of a light wavelength. The light wavelength may be for example in a spectral range of 600 to 1500 nm. In this spectral range typical characteristics of different endogenous contents of a human or animal tissue can be detected. Accordingly, it becomes possible to detect a particular endogenous content in the medium based on its spectral characteristics.

. The light diffusion characteristic of the medium may be based on an attenuation of photons passing the medium.

One advantage of a light absorption characteristic and/or light scattering characteristic is that it may be obtained fast and without impact such as harm to the medium.

In one aspect, the method may further comprise an operation of determining a biological characteristic of the medium as a function of the optical characteristic, for example as a function of spectral characteristics of the medium, as explained above. The medical imaging parameter may be adjusted as a function of the biological characteristic.

For example, the biological characteristic may comprise a density of the medium. For example, it may be that a fatty medium (i.e. comprising an increased amount of lipid in relation to its liquid content, in particular water) is less dense than a lean medium. In other words, a less dense medium may be characterized by comprising a relatively high proportion of lipids and/or a relatively low proportion of liquids. On the other hand, it may be that a liquid-rich (i.e. water-rich) medium (i.e. comprising a relatively low amount of lipid in relation to its water content) is denser than a fatty medium. In other words, a dense medium may be characterized by comprising a relatively low proportion of lipids and/or a relatively high proportion of liquids. Accordingly, by determining the amount of lipid in the medium (based on the optical characteristic), the density of the medium may be determined. The density may also depend on other factors such as the amount of bones, liquid such as blood in vessels, collagen and/or hemoglobin in the medium.

One advantage of determining a biological characteristic of the medium is that the quality of the medical imaging data may be improved. For example, an ultrasound imaging parameter may be adjusted as a function of the density of the medium. Furthermore, the biological characteristic may be used as an additional marker for examining the medium. For example, the biological characteristic may determine whether a tissue is healthy or sick.

In one aspect, the biological characteristic may comprise a characteristic of an endogenous content of the medium.

The endogenous content may comprise information about endogenous substances in the medium. For example, the endogenous content may comprise information about a biological tissue. Furthermore, the endogenous content may for example comprise a lipids characteristic. A lipid characteristic may comprise information about a degree and/or volume and/or amount and/or rate of lipids in the medium. A lipid characteristic may also comprise a qualitative determination, for example, a qualitative determination whether a medium is lipids or not. A qualitative determination may also determine whether a medium contain an unusually high amount of lipids.

The endogenous content may comprise information about oxigenated and/or non-oxigenated hemoglobin. The endogenous content may also comprise information about other blood related parameters, such as the relative amount of blood in the medium and/or of an oxygen saturation rate or level of the medium (for instance defined by the relation between oxigenated and non-oxigenated hemoglobin).

The endogenous content may comprise information about collagen characteristics. Collagen characteristics may be used as a marker for the elasticity. Therefore, collagen characteristics may be used for example for optimizing a ShearWave^{™} Elastography (SWE) method. An exemplary SWE method is described in EP1546757B1 of the same applicant.

One advantage of a characteristic comprising an endogenous content of the medium is that it may improve the image quality of medical imaging methods. For example, medical imaging parameters of Pulsed-Wave-Doppler may be better adjusted to a particular medium.

In one aspect, the medical imaging parameter may comprise at least one of: an ultrasound imaging parameter, a tomography imaging parameter, a three-dimensional (3D) imaging parameter, a slicing coefficient in a 3D imaging method, an X-ray imaging parameter, a mammography imaging parameter, and/or magnetic resonance imaging (MRI).

For example, the 3D image may be compiled based on a plurality of 2D images. More in particular, for example, a 3D-ROI may be determined. Thus, it may be useful to adapt a slicing strategy (using the slicing coefficient) based on the optical characteristic to obtain a suitable 3D image of the ROI.

The X-ray imaging parameter may comprise information about for example an X-ray doses and/or X-ray degree.

One advantage of this approach is that an ultrasound method and optical acquisition may be easily combined. For example, the optical acquisition may be conducted with an ultrasound imaging system. The ultrasound imaging system may comprise an optical element (for example an optical sensor) to obtain the optical characteristic. For example, the optical element may be included in a probe of the ultrasound imaging system, in particular the probe which also contains ultrasound transducer element(s). In this case, it is advantageously fast and easy for a user to reliably acquire the optical characteristic and the medical data of the same area of a medium.

In one aspect, the medical imaging parameter may comprise a data acquisition parameter defining a medical data acquisition method. Furthermore, the method may also comprise a data processing parameter defining a medical data processing method.

The data acquisition parameter may define a medical data acquisition method. A medical data acquisition method may be, for example, an ultrasound insonification/acquisition method. The medical data acquisition method may precede a medical data processing method. The medical data acquisition method may, for example, consist of: acquire raw data of the medium, which may be processed by the medical data processing method to construct medical image data.

The medical data processing method may process the obtained or acquired medical image data. For example, the medical data processing method may be a beamforming method.

The optical acquisition and medical data acquisitions may be simultaneous or at different time. It is also possible that the optical acquisition is before the medical data acquisition, to allow the use of the optical characteristic to configure the medical data acquisition.

It is also possible, that the medical data acquisition may be done before, as the optical characteristic may only be used to (post-) process the imaging data. For example, the optical characteristic may be used to adjust the speed of sound in a processing method such as beamforming.

In one aspect, the data acquisition parameter may comprise at least one of: an ultrasound wave coefficient characterizing an ultrasound wave transmitted into the medium, and/or a radiation doses of X-ray waves transmitted into the medium. The data processing parameter may comprise a speed of sound of the medium, an ultrasound attenuation coefficient of the medium, and/or an ultrasound scattering and/or absorption coefficient.

This may in particular be the case if the medical acquisition method is an ultrasound insonification/acquisition method.

The ultrasound wave coefficient may be or may comprise, for example, a frequency of transmitted ultrasound waves. For example, in case an increased optical absorption in the medium is deduced from the obtained optical characteristic, a reduced frequency of transmitted ultrasound waves may be chosen because ultrasound waves with reduced frequency are less attenuated.

Furthermore, the ultrasound wave coefficient may comprise an ultrasound stimulation wave coefficient of stimulation waves transmitted into the medium. The ultrasound wave coefficient may be SWE stimulation pulses which cause a shear wave in the medium, i.e. so-called "push pulses", for example as described in EP 1546757 B1 of the same applicant. In case of an increased absorption, a shorter push pulse may be used. Correspondingly, in case of an increased scattering, a longer push may be used. For example, a fatty medium with a high degree of lipids may lead to a high viscosity and thus to a low elasticity. Therefore, it may be advantageous to adapt SWE parameters. For example, a rheological model, for example Voigt's model, may be used to adapt the SWE processing or elasticity estimation of the fat characteristic, as also described in for example: S. Catheline et.al. (2005). Measurement of viscoelastic properties of homogeneous soft solid using transient elastography: An inverse problem approach. The Journal of the Acoustical Society of America. 116. 3734-41. 10.1121/1.1815075.

In one aspect, the medical imaging parameter may be a data processing parameter, which is adjusted to match more precisely a real characteristic of the medium. Furthermore, the medical imaging parameter may be a data processing parameter, which is adjusted, such that a model used in the medical data processing method matches more precisely a real characteristic of the medium.

As an example, a data processing parameter may be the speed of sound. The speed of sound may be adjusted to the real speed of sound, as determined based on the biological and physiological characteristics of the medium.

The adjustment of a processing parameter may, for example, depend on the degree of fat (i.e. lipids concentration) in the body. It is possible that a mathematical model may be used which takes into account the viscosity and not only elasticity. A mere elasticity model would not precisely reflect the viscous characteristic of the fatty medium. An elasticity model only takes into account the stiffness but not the viscosity. In contrast, a viscosity model may take into account viscosity and elasticity. Therefore, an adaptation of the used model may be made as a function of the biological characteristic of the medium (i.e. in the mentioned example the degree of fat). The adjustment of a processing parameter may only influence the medical data processing method but not the medical data acquisition method.

In one aspect, the medical imaging parameter may be obtained in a medical imaging method. The method may further comprise combining at least two medical imaging methods using the optical characteristic to adjust at least one medical imaging parameter of at least a first one of the two medical imaging methods.

In other words, the medical imaging parameter may be used to configure a medical imaging method.

Examples of medical imaging methods may be ultrasound imaging, tomography and/or three-dimensional (3D) imaging, X-ray imaging, mammography imaging, and magnetic resonance imaging (MRI).

In one example, the X-ray doses or X-ray degree may be configured based on the optical characteristic, i.e. adapted to the specific medium. In another example, the length of a push pulse in SWE may be configured based on the optical characteristic.

A combination of two medical imaging methods may be, for example, the combination of ultrasound imaging and mammography. In this example, optics might serve as a bridging modality between ultrasound imaging and mammography.

One advantage of this aspect is that the advantages of at least two medical imaging methods may be combined. It may even be that there is a synergic effect by combining at least two medical methods, as explained in the following exemplary aspect.

In one aspect, the medical imaging parameter may be a data processing parameter, which is adjusted such that the image data of the first medical imaging method more precisely match with anatomic and/or geometric characteristics of the medium, or other characteristics of the medium, for example the speed of sound in the medium. Such a characteristic defined by the medium, for example the speed of sound, may be an average characteristic for the complete medium or a region of interest in the medium. For instance, an average speed of sound across a region of interest in the medium may be determined, or several values for different regions of interest or for different parts of a region of interest.

In other words, the medical imaging parameter may be used to configure a medical data processing method, such as for example a beamforming method.

For example, a data processing parameter, such as the speed of sound, may be adjusted such that the anatomic reconstruction is more precise. Accordingly, a more reliable volumetric or geometric matching between images, for example of a ROI in a medium, of different medical imaging methods, for example ultrasound imaging and mammography, may be achieved.

In one aspect, the method may comprise selecting and/or confirming a medical imaging method as a function of, the optical characteristic. The medical imaging parameter of the selected and/or confirmed medical imaging method may be adjusted as a function of the optical characteristic.

For example, the optical characteristic may be used to determine the density of the medium. A breast considered to have a 'high density' may require ultrasound imaging after or in place of a mammography for confirmation of the diagnostic. Therefore, in case of a fatty (i.e. less dense) breast, mammography may be used and the X-ray doses may be adapted. Furthermore, in case of a not fatty or lean (i.e. dense) medium, ultrasound imaging may be used and the stimulation waves of SWE may be adapted (mammography may optionally be omitted in this case).

The present disclosure may also refer to a medical data acquisition method. The medical data acquisition method may comprise an adjusting a medical imaging parameter according to any examples of the present disclosure and obtaining medical data of the medium as a function of the adjusted medical imaging parameter.

The obtained medical data may also be referred to as raw data.

One advantage of the disclosed medical data acquisition method is an easier image quality, due to the adjusted medical imaging parameter.

The present disclosure may also refer to a method of processing medical image data. The method of processing medical image data may comprise adjusting a medical imaging parameter according to any examples of the present disclosure, and processing medical image, data as a function of the adjusted medical imaging parameter.

One advantage of this disclosed method is that the method of processing medical image data may be conducted independently from other methods. For example, the method of processing medical image data may be conducted independently from the medical data acquisition.

The present disclosure may also refer to a medical imaging method. The medical imaging method may comprise a medical data acquisition method and a method of processing medical image data according to any examples of the present disclosure.

As an example, the medical imaging method may hence comprise the medical data acquisition method to obtain raw data, and the medical data processing method to process the raw data, in order to obtain image data. Those image data may then be displayed on dedicated screens or any other display device having the required specifications. At least one of the two sub-methods may use an adjusted parameter, as defined according to any examples of the present disclosure.

Furthermore, the medical imaging data may, for example, comprise the medical data acquisition method, and the medical data processing method, as described according to any examples of the present disclosure.

In one aspect, adjusting the medical imaging parameter and/or configuring at least one of the medical imaging methods and the medical data processing method may be performed in real-time.

One advantage of this aspect is that the method may improve the examination speed and the image quality. For example, a real-time performance of adjusting the medical imaging parameter may comprise an iterative process to improve the medical imaging parameter and thus obtain a better image quality.

The present disclosure may also refer to a computer program comprising computer-readable instructions which when executed by a data processing system cause the data processing system to carry out a method according to any examples of the present disclosure. In case the method operations may comprise any (physical) aspects which go beyond a mere data processing (for example an ultrasound wave emission), the computer program may further comprise computer-readable instructions which when executed by a data processing system cause any external elements of a system (for example an ultrasound transducer device) to carry out these operations.

The disclosure further refers to a system for adjusting a medical imaging parameter of a medium. The system comprises a processing unit, which is configured to obtain an optical characteristic of the medium and to adjust the medical imaging parameter as a function of the optical characteristic.

One advantage of a system for adjusting a medical imaging parameter of a medium may be a compact design. For example, elements of the system configured to obtain an optical characteristic of the medium and to adjust the medical imaging parameter as a function of the optical characteristic may be arranged in one compact design, such as a housing or a probe. Furthermore, the elements may be arranged to scan the same region in a medium, such that the optical characteristic may be assigned to the acquired medical data. It is however also possible that at least one of the two elements is not physically part of the system, optionally based in another place (i.e. external to the system) and only provides the respective data (i.e. medical data or the optical characteristic) to the system.

In one aspect, the system may be a multi-modal system comprising a plurality of different medical imaging modes. Adjusting the medical imaging parameter as a function of the optical characteristic may comprise selecting and/or confirming a medical imaging mode as a function of the optical characteristic.

A bimodal system may be configured to acquire medical data and optical characteristic of a medium. A multi-modal system may be configured to acquire at least two different types of medical data (for example ultrasound data and mammography data) and optionally also an optical characteristic. The optical characteristic may however also be obtained by another system or device.

"Adjusting" may also mean selecting the best mode in a multi-modal system.

One advantage of this aspect is that the system may automatically select or propose to the user the estimated best medical imaging mode. For example, the examiner may use a multi-modal system comprising ultrasound imaging and mammography for examination and the system automatically may select ultrasound as the best mode for the specific examination conditions.

The system may furthermore be configured to perform any of the method features or operations described above.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

### BRIEF DESCRIPTION OF THE DRAWINGS

Fig. 1 shows a flowchart of a method 100 for adjusting a medical imaging parameter of a medium according to examples of the present disclosure.
Fig. 2A shows a schematic drawing of a medical imaging system 200 according to examples of the present disclosure.
Fig. 2B shows a schematic drawing of an ultrasound imaging system 10 according to examples of the present disclosure.
Fig. 3 shows overview of the structure of the medical imaging parameter according to examples of the present disclosure.
Fig. 4 shows a flowchart of a medical imaging method 400 according to examples of the present disclosure.
Fig. 5 shows a flowchart of a medical data acquisition method 500 according to examples of the present disclosure.
Fig. 6 shows a flowchart of a method of processing medical image data 600 according to examples of the present disclosure.
Fig. 7A shows the absorption spectra of different biological materials.
Fig. 7B shows the speed of sound for different densities.

### DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

In general, conventional medical imaging focuses on examining a medium by transmitting and receiving one mode of signal. However, combining different modes may improve medical imaging and help to get faster and more accurate examination results.

WO 2020178522 A2 describes a bimodal ultrasonic probe comprising an optical device for diagnosis. The portable bimodal probe intended to be applied to a biological tissue to be examined. The probe comprises an ultrasonic transducer configured to transmit ultrasonic waves into the tissue and to receive ultrasonic waves reflected by the tissue, the transducer extending along a transverse axis. Furthermore, the probe comprises at least two optodes arranged on either side of the transverse axis, such that the transducer extends between the two optodes. Each optode comprises a light transmitter configured to transmit a light wave to the tissue and an optical detector configured to detect a light wave propagated by the tissue. The optodes are designed such that at least one light transmitter and at least one optical detector are arranged on either side of the transducer. At least one optical detector has a detection surface formed of a semiconductor material and connected to an electronic card.

The system and method described herein are related to technologies for optimizing a process for constructing image data of a medium, in particular in the context of medical imaging. The method is in particular suitable for processing signal data of a medium scanned by a bimodal device. The method may be used in a device such as for instance an ultrasound system.

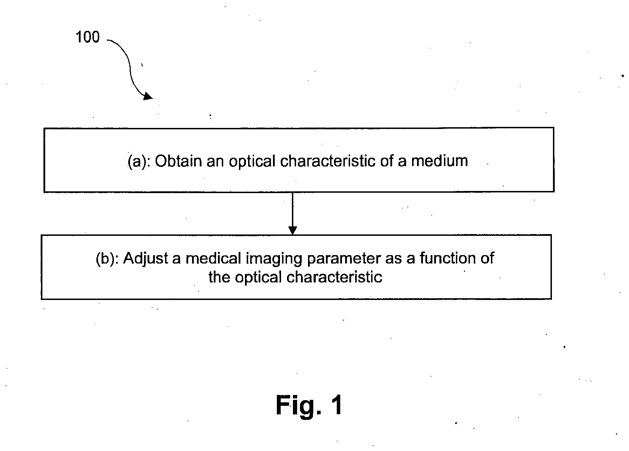

Fig. 1 shows a flowchart of a method 100 of adjusting a medical imaging parameter of a medium according to the present disclosure. The method comprises an operation (a) of obtaining an optical characteristic of the medium. The optical characteristic may comprise a light absorption and the medium may be a human or animal tissue or any material to be examined. The optical characteristic may be acquired using for example an optical element as described below. However, the optical characteristic may also be obtained from a data storage or a data interface, i.e. may be acquired by any kind of optical element beforehand and/or remotely.

Furthermore, the method comprises an operation (b) of adjusting the medical imaging parameter as a function of the optical characteristic. The medical imaging parameter may comprise a tomography and/or three-dimensional (3D) imaging parameter.

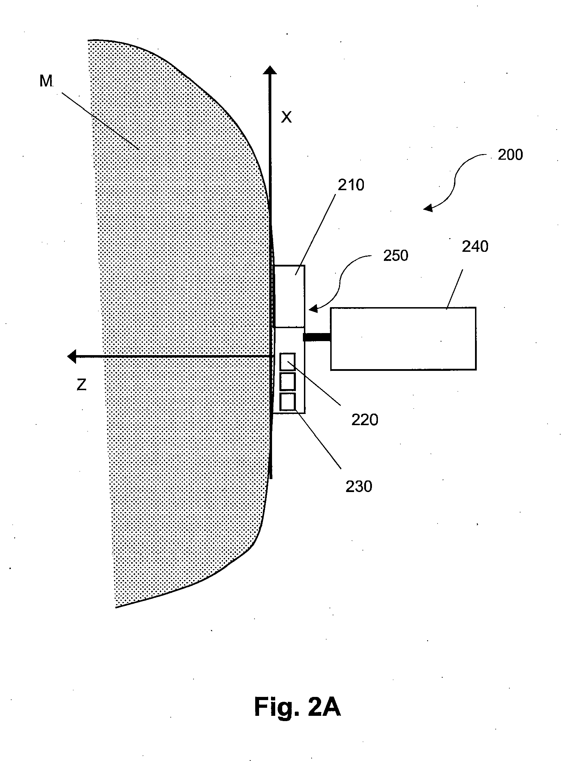

Fig. 2A shows a schematic drawing of a medical imaging system 200 according to examples of the present disclosure. The system 200 shown on Fig. 2A is adapted for adjusting a medical imaging parameter of a medium M. The medium M may be for instance living tissues and/or in particular human tissues of a person.

The system 200 may be any medical imaging or sensor device. The system 200 may be for instance a device for tomography, X-ray, mammography or magnetic resonance imaging (MRI). Accordingly, the medical imaging parameter may comprise an imaging parameter related to the system 200.

The system 200 may for example be or comprise a device for ultrasound imaging.

The system 200 may include for instance:
- An optical element 210. The optical element 210 may be configured to transmit a light pulse into the medium M and/or to receive a plurality of signals from the medium M, optionally in response to transmitting the pulse into the medium M. The optical element may be comprised by a probe 250 of the system or may be external thereto.
- a transducer element 220. The transducer element 220 may be configured to transmit a pulse into the medium M and/or to receive a plurality of signals from the medium M, optionally in response to transmitting the pulse into the medium M. The transducer element 220 may be comprised by the probe 250 of the system.
   Instead of a single transducer element 220, the system may also comprise a transducer array 230 comprising a plurality of transducer elements 220. For example, a linear array 231 may be provided typically including a plurality transducer elements 220 (for instance 100 to 300 or more, in particular 126, 256, 512 or 1024 transducer elements) juxtaposed along an axis X (horizontal or array direction X). In this illustrative example, the transducer array 220 is adapted to perform a bidimensional (2D) imaging of the medium M, but the transducer array 220 could also be a bidimensional array adapted to perform a 3D imaging of the medium M. Accordingly, a matrix of transducers may be used. It is though also possible that the system comprises a single line of transducer arrays 230 moveable in a probe, such that 3D imaging data may be constructed. The transducer array 230 may also be a convex array including a plurality of transducer elements 220 aligned along a curved line. The same transducer element(s) 220 may be used to transmit a pulse and receive the response, or different transducer elements 220 are used for transmission and reception. There may be one or more emitting transducer elements 220 and a plurality of receiving transducer elements 220. In a further alternative, only a single transducer element 220 may be used which receives a plurality of signals which have different spatial properties (for example originating from different spatial regions). The transducer element 220 may for instance be electronically or physically moveable. The transducer element 220 and/or the transducer array 230 may be part of the probe 250 of the system.
- a processing unit 240 controlling the optical element 210, the transducer element 220 or the transducer array 230. The processing unit 240 may acquire signals from the transducer element 220 or the transducer array 230. The processing unit 240 may be part of a probe which may also comprise at least one transducer element 220. Alternatively, the processing unit 240 may be external to the probe, or consist of several devices which are partially part of the probe and partially external thereto. The probe 250 may be connectable to processing unit 230 via a cable or via a wireless communication interface. In other words, the probe 250 may be configured to be operated wirelessly. For example, the probe 250 may in this case comprise a battery to be operated autonomously.
   The processing unit 240 may comprise receiving devices for amplifying and/or filtering signals received from the probe 250, and converters (analog to digital converters and digital to analog converters) for transforming the signals into data representing the signal. The data may be stored into a memory in the processing unit or directly processed for calculating intermediate processed data (beamformed data). The processing unit 250 may use any known processing method to process the image on the bases of the signals received from the probe, such as beamforming. The processed ultrasound image data may be:
   - a simple medium image (B-mode image) usually in grey scale for visualizing organs inside the medium, or
   - an image showing velocity or flow in the medium (colour image) for example useful for visualizing blood vessels in the medium, or
   - an image showing a mechanical characteristic of the medium (elasticity) for example useful for identifying tumours inside the medium (for example ShearWave^{™} Elastography (SWE) image data as described above).

The processing unit 240 may send data to an external device, such as for example, a server, a computer on which an artificial intelligence (AI) algorithm may be running, a dedicated workstation, presenting data, a device for displaying images obtained from the processing unit or any of the other external devices. The processing unit 240 may be configured to obtain an optical characteristic of the medium and adjust the medical imaging parameter as a function of the optical characteristic. Accordingly, the method according to the present disclosure, in particular a method of adjusting a medical imaging parameter of a medium, may be carried out by the processing unit 240 or any external device. Furthermore, the process for constructing the image data may be carried out by the same processing unit 240 as that one for optimizing the optimizing a process, or (at least in part) by another one or several others.

According to further examples, the system 200 may include at least one processor and memory. In examples, the processor and memory unit may be incorporated into the system 200 or may be a computer or computer communicatively linked thereto. Depending on the exact configuration and type of computing device, memory (storing, instructions to evaluate imaging data or otherwise perform the methods described herein) may be volatile (such as RAM), non-volatile (such as RAM, flash memory, etc.), or some combination of the two. Further, the system 200 may also include storage devices (removable and/or non-removable) including, but not limited to, magnetic or optical disks or tape. Similarly, the system 200 may also have input device(s) such as keyboard, mouse, pen, voice input, etc. and/or output device(s) such as a display, speakers, printer, etc. Also included in the environment may be one or more communication connections, such as LAN, WAN, point to point, etc. In embodiments, the connections may be operable to facility point-to-point communications, connection-oriented communications, connectionless communications, etc.

The system 200 may typically include at least some form of computer readable media. Computer readable media may be any available media that may be accessed by processing unit (or processor) or other devices comprising the operating environment. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The processing unit 240 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media. Such networking environments are commonplace in offices, enterprise-wide computer networks, intranets and the Internet.

The optical element 210 may comprise borescopes, fiberscopes, fiber optic faceplates, lenses, prisms, mirrors, light-emitting diodes, photodetectors, optical fibers, fiber optic cables, and/or fiber optic bundles.

The optical element 210 may be configured to generate and/or record and/or receive signals. The optical element 210 and/or the processing unit 240 may be configured to obtain an optical characteristic of the medium M.

The optical element 210 may for example comprise a laser printed circuit board (PCB) and edge emitting diodes (not shown in fig. 2A). The edge emitting diodes may emit a laser beam. Furthermore, the optical element may comprise an optical window, a prism, a detector PCB, and a detector (not shown in fig. 2A). The detector may detect any light, in particular reflections of the laser beam, which was emitted by the edge emitting diodes.

The transducer element 220 may comprise piezo-crystals and/or other components that may be configured to generate and/or record and/or receive signals. The terms transducer and transducer elements may be used synonymously throughout this disclosure unless denoted differently.

Accordingly, in one example the system 200 may use ultrasound waves, the transducer elements being then adapted to ultrasound waves. The method optionally may produce ultrasound images of the medium M and/or may send data to a dedicated server or working station.

The system 200 may be configured to acquire more than one type of medical data, for example not only ultrasound data, but also mammography data (not shown in fig. 2a). In other words, the system may also be a multimodal system.

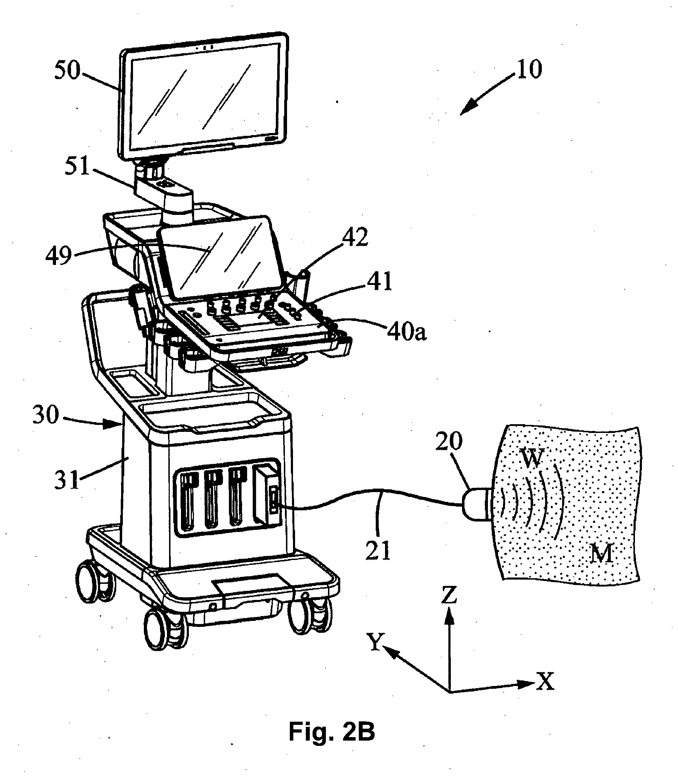

Fig. 2B shows a schematic drawing of an ultrasound imaging system 10 according to examples of the present disclosure. The ultrasound imaging system 10 may be an example of a medical imaging system 200, as described in context of. fig. 1A.

The ultrasound imaging system 10 may comprise:
- a probe 20 (for example corresponding to the probe 250 of fig. 2A),
- a processing unit 30 for processing an image on the bases of signals received by the probe (for example corresponding to the processing unit 240 of fig. 2A),
- a control panel 40 connected to the processing unit, said control panel at least comprising buttons 41 and a touch pad 42, and
- a display 50 for visualizing the image.

The probe 20 may be connected to the processing unit 30 via a cable 21 or via a wireless connection, and it is able to emit ultrasound waves W into a medium M and to receive ultrasound waves W from the medium M, said received ultrasound waves being consequent or resulting from reflections of said emitted ultrasound waves on diffusing particles inside said medium. The probe 20 may be a transducer array comprising a plurality of transducers, each one converting an electric signal into a vibration and reciprocally. A transducer is for example a piezoelectric element. The transducer array may comprise hundred transducers or more. The transducer array is a linear or curved and is disposed on an outer surface of the medium M so as to be coupled to the medium and to vibrate and to emit or receive ultrasound waves W.

As further described in context of fig. 2A, the probe 20 may further comprise an optical element configured to obtain an optical characteristic of the medium. However, it is also possible that the optical characteristic is obtained by another device comprised by, connectable to, or associated with the system 10. For example, the system may comprise or be connectable to a distinctive optical sensor, which may for example be attached to the probe. The optical sensor may be connected to the system via a cable or via a wireless connection. The optical characteristic may also be obtained by any device which is external and/or remote to the system 10, as also described in context of fig. 2A.

The processing unit 30 may comprise receiving devices for amplifying and/or filtering signals received from the probe 20, and converters (analog to digital converters and digital to analog converters) for transforming the signals into data representing the signal. The data may be stored into a memory in the processing unit or directly processed for calculating intermediate processed data (beamformed data). The processing unit 30 may use any known processing method to process the image on the bases of the signals received from the probe, such as beamforming. The processed ultrasound image data may be:
- a simple medium image (B-mode image) usually in grey scale for visualizing organs inside the medium, or
- an image showing velocity or flow in the medium (colour image) for example useful for visualizing blood vessels in the medium, or
- an image showing a mechanical characteristic of the medium (elasticity) for example useful for identifying tumours inside the medium (for example ShearWave^{™} Elastography (SWE) image data as described above).

The display screen 50 may be a screen for visualizing the image processed by the processing unit 30. The display 50 may also visualize other information such as scales used in the image, or configuration information for the processing or any information such as help information or contextual gesture help for the touch pad 42.

The display screen may by articulated on a support arm 51 for better positioning for the user. The display screen is usually a screen of a great size (at least 20 inches) for better image visualization to the user.

The control panel 40a is for example a portion of the system casing 31, said portion comprising a panel casing having a substantially flat surface inclined towards the user for manipulation by one hand of said user. The control panel 40a may be moved by a hanger upwards and downward for being adapted to the user size, and may be optionally moved frontward and rearward for being adapted to the user position. As seen on figure 1, the control panel 40a may include a control panel display screen 49 for visualizing several configuration information. This control panel display screen 49 can also be hinged to the control panel casing 48 for being inclined into a different inclination than the control panel surface.

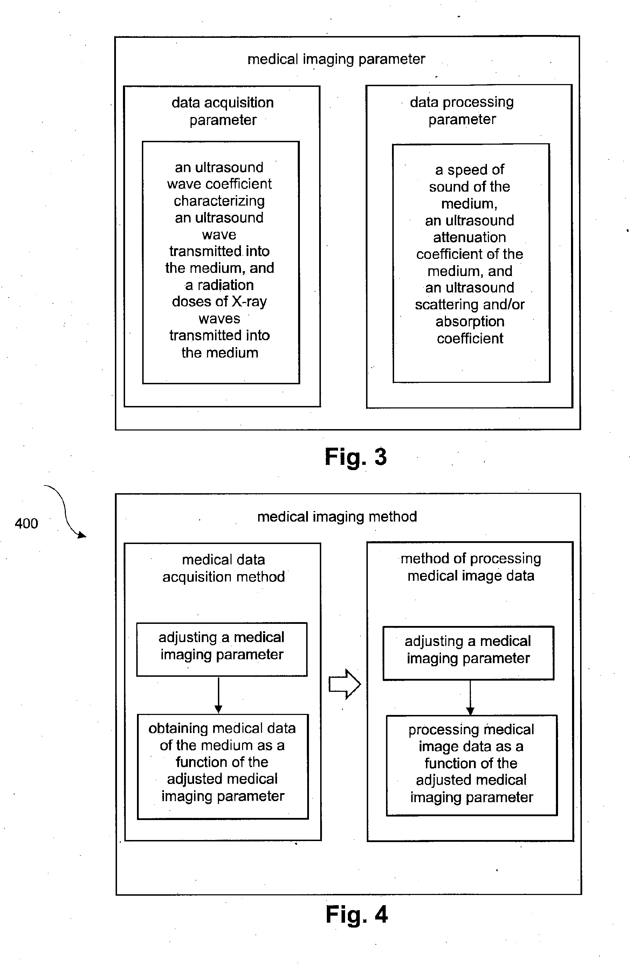

Fig. 3 gives an overview of the structure of the medical imaging parameter according to examples of the present disclosure. The medical imaging parameter comprises a data acquisition parameter and a data processing parameter.

The data acquisition parameter may define a medical data acquisition method. The data acquisition parameter may comprise for example an ultrasound wave coefficient characterizing an ultrasound wave transmitted into the medium, and/or a radiation doses of X-ray waves transmitted into the medium. Accordingly, the data acquisition parameter may influence the way how medical data are obtained from the medium, for example, how the medium is insonified by imaging waves.

Alternatively, or additionally, the data processing parameter may define a medical data processing method. The data processing parameter may comprise for example at least one of: a speed of sound of the medium, an ultrasound attenuation coefficient of the medium, and an ultrasound scattering and/or absorption coefficient.

Furthermore, the medical imaging parameter may comprise at least one of: an ultrasound imaging parameter, a tomography imaging parameter, a three-dimensional (3D) imaging parameter, a slicing coefficient in a 3D imaging method, an X-ray imaging parameter, a mammography imaging parameter, and magnetic resonance imaging (MRI).

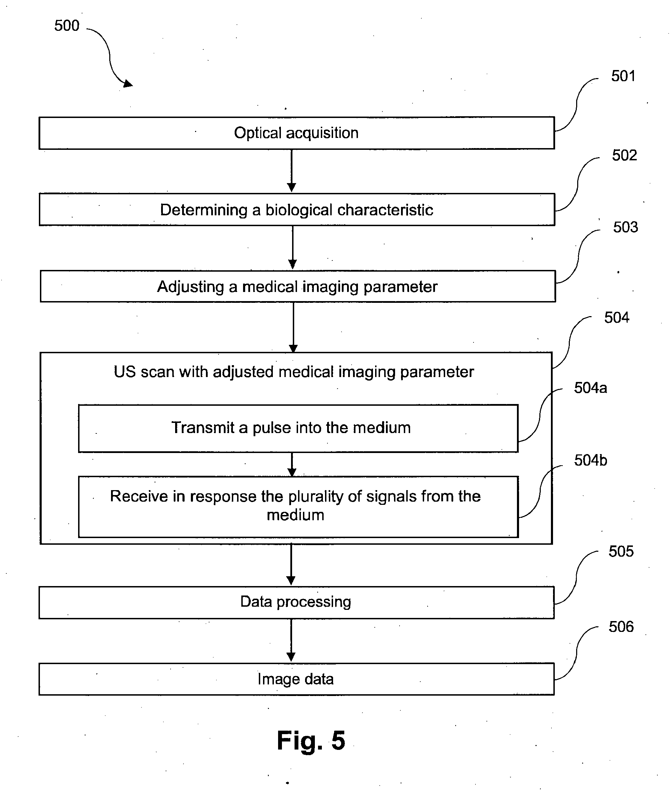

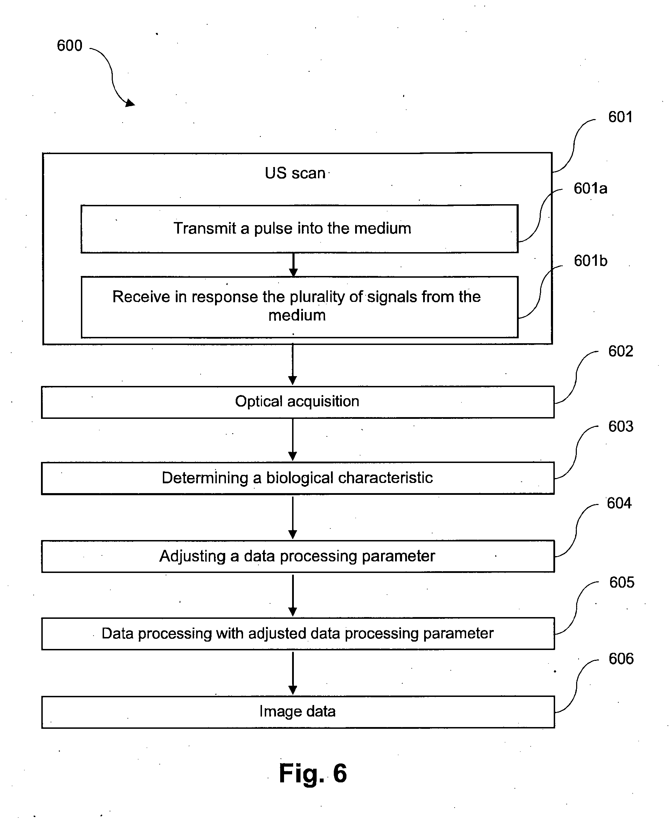

Fig. 4 shows a flowchart of a medical imaging method 400 according to examples of the present disclosure. The medical imaging method may comprise a medical data acquisition method and a method of processing medical image data. The medical data acquisition method may comprise adjusting a medical imaging parameter and obtaining medical data of the medium as a function of the adjusted medical imaging parameter. Additionally or alternatively, the method of processing medical image data may comprise adjusting a medical imaging parameter and processing medical image data as a function of the adjusted medical imaging parameter. The medical data acquisition method may precede the method of processing medical image data. In particular the acquired medical data (i.e. the output of the medical data acquisition method) may be used as an input in method of processing medical image data. Examples of medical imaging methods are shown in more detail in figures 5 and 6.

Fig. 5 shows a flowchart of a medical data acquisition method 500 according to examples of the present disclosure.

In a first operation 501, an optical acquisition may be conducted, for example using an optical element as described above. The optical characteristic may however also be obtained from a data storage or a data interface, i.e. may be acquired by any kind of optical element beforehand and/or remotely. A second operation 502 may comprise determining a biological characteristic. In other words, based on the optical characteristic, a biological characteristic, for example the endogenous content of the medium may be determined and thus for example an ultrasound wave absorption characteristic of the medium. A third operation 503 comprises the adjusting of a medical imaging parameter, for example using a processing unit. In a fourth operation 504, an ultrasound scan with adjusted medical parameter is conducted. The ultrasound scan may comprise transmitting a pulse 504a into the medium. Furthermore, in response to the transmitted pulse, the ultrasound scan may comprise receiving a plurality of signals 504b from the medium. The ultrasound scan may also be in the form of any specific ultrasound imaging mode, like for example a B-mode (brightness mode), a doppler mode, or ShearWave^{™} Elastography (SWE) Smode (for example as described in EP 1546757 B1 of the same applicant). The ultrasound scan may also be carried out by any external device, for example remotely to the processing unit.

In one example, the medical imaging parameter may be an ultrasound stimulation wave coefficient of so-called "push pulses" in a SWE mode, as described above. In case of an increased absorption characteristic, a shorter push pulse may be emitted. Correspondingly, in case of an increased scattering, a longer push may be used.

In a fifth operation 505 the acquired ultrasound data may be processed, for example in order to construct image data. In the optional operation 506, image data is obtained, i.e. as an output of the operation 605.

Fig. 6 shows a flowchart of a method of processing medical image data 600 according to examples of the present disclosure. The method of fig. 6 may principally correspond to the method of fig. 5. However, in addition or as alternative, the data processing operation may be adapted based on the optical characteristic (i.e. in addition or as an alternative to an adaption of the ultrasound scan operation). Moreover, in the method of fig. 6, the order of the operations may differ from that one in the method of fig. 5 (as shown in fig. 6) or may be similar to the order of the operations of method of fig. 5

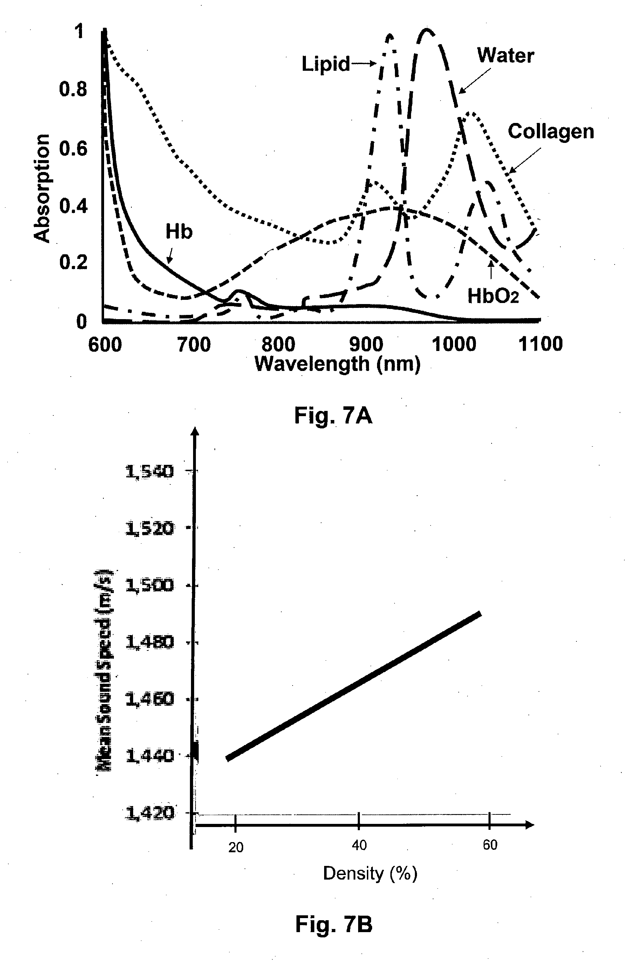

In a first operation 601, a ultrasound scan may be conducted. The ultrasound scan may comprise transmitting a pulse into the medium 601a and receiving in response the plurality of signals from the medium 601b. However, operation 601 is merely optional, as the data may also be obtained from any external device, for example from a data storage device. A second operation 602 may comprise obtaining an optical characteristic. Again, operation 602 is merely optional, as the optical characteristic may also be obtained from any external device, for example from a data storage device. In a third operation 603, determining of a biological characteristic may be conducted. A fourth operation 604 may comprise adjusting a data processing parameter. A fifth operation 605 may comprise data processing with an adjusted data processing parameter. In optional operation 606, image data may be obtained, i.e. as an output of the operation 605. A more specific example of the method of fig. 6 is described in the following with reference to fig. 7A and 7B.

Fig. 7A shows the absorption spectra of different biological materials.

Biological material like lipid typically has a maximal absorption at a wavelength of about 920 nm. Water at a temperature of 37 °C (usual temperature of an alive human body) has typically a maximal absorption at a wavelength of about 980nm. The biological characteristics, i.e. the endogenous content of the medium, may thus be derived from a measured spectrum of the medium. For example, a high absorption of light at a wavelength of 920 nm may indicate a high content of lipids in the considered medium. This approach is especially effective in the field of breast examinations. A less dense breast usually comprises relatively high amount of lipid in relation to water, while a dense breast comprises a relatively water-rich tissue in relation to lipid what can precisely be determined based on the spectrum.

In other words, the density of a medium may be determined based on optical characteristics, i.e. absorption spectrum at one or several wavelengths (cf. operations 602 and 603 in fig. 6).

Fig. 7B shows the speed of sound for different densities. In general, a correlation between speed of sound and the density of a medium may be detected. For example, it may be that a high speed of sound indicates a high density of the medium.

Accordingly, the speed of sound may be determined based on the density (cf. operation 604 in fig. 6). For example, if the density of medium was determined based on the absorption spectrum, the speed of sound in the medium may be determined using Fig. 7B.

The adjusted speed of sound may then be used in a medical data processing method, i.e. in a respective beamforming method to construct more precise ultrasound image data (cf. operation 605 in fig. 6).

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially", and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A method of adjusting a medical imaging parameter of a medium,
wherein the method comprises:
obtaining an optical characteristic of the medium,
adjusting the medical imaging parameter as a function of the optical characteristic.

2. The method according to claim 1, wherein
the optical characteristic comprises a light absorption characteristic and/or a light scattering characteristic of the medium.

3. The method according to claim 1 or 2, wherein
the method further comprises:
determining a biological characteristic of the medium as a function of the optical characteristic, wherein
the medical imaging parameter is adjusted as a function of the biological characteristic.

4. The method according to the preceding claim, wherein
the biological characteristic comprises a characteristic of an endogenous content of the medium.

5. The method according to any one of the preceding claims, wherein
the medical imaging parameter comprises at least one of:
an ultrasound imaging parameter,
a tomography imaging parameter,
a three-dimensional (3D) imaging parameter,
a slicing coefficient in a 3D imaging method,
an X-ray imaging parameter,
a mammography imaging parameter, and
magnetic resonance imaging (MRI).

6. The method according to any one of the preceding claims, wherein
the medical imaging parameter comprises at least one of:
a data acquisition parameter defining a medical data acquisition method, and
a data processing parameter defining a medical data processing, method.

7. The method according to the preceding claim, wherein
the data acquisition parameter comprises at least one of:
an ultrasound wave coefficient characterizing an ultrasound wave transmitted into the medium, and
a radiation doses of X-ray waves transmitted into the medium,
and/or
the data processing parameter comprises at least one of:
a speed of sound of the medium,
an ultrasound attenuation coefficient of the medium, and
an ultrasound scattering and/or absorption coefficient.

8. The method according to the preceding claim, wherein
the medical imaging parameter being a data processing parameter is adjusted to match more precisely a real characteristic of the medium, and/or
the medical imaging parameter being a data processing parameter is adjusted, such that a model used in the medical data processing method matches more precisely a real characteristic of the medium.

9. The method according to any one of the preceding claims, wherein
the medical imaging parameter is obtained in a medical imaging method, wherein the method further comprises:
combining at least two medical imaging methods using the optical characteristic to adjust at least one medical imaging parameter of at least a first one of the two medical imaging methods.

10. The method according to the preceding claim, wherein
the medical imaging parameter being a data processing parameter is adjusted such that the image data of the first medical imaging method more precisely match with anatomic and/or geometric characteristics of the medium.

11. The method according to any one of the preceding claims, further comprising:
selecting and/or confirming a medical imaging method as a function of the optical characteristic, wherein
a medical imaging parameter of the selected and/or confirmed medical imaging method is adjusted as a function of the optical characteristic.

12. A medical data acquisition method, comprising:
adjusting a medical imaging parameter according to the method of any one of the preceding claims,
obtaining medical data of the medium as a function of the adjusted medical imaging parameter.

13. A method of processing medical image data, comprising:
adjusting a medical imaging parameter according to the method of any one of the preceding claims,
processing medical image data as a function of the adjusted medical imaging parameter.

14. A medical imaging method, comprising at least one of:
the medical data acquisition method of claim 12, and
the method of processing medical image data of claim 13.

15. The method according to the preceding claim, further comprising:
adjusting the medical imaging parameter and/or configuring at least one of the medical imaging method and the medical data processing method is performed in real-time.

16. A computer program comprising computer-readable instructions which when executed by a data processing system cause the data processing system to carry out the method according to any one of preceding claims.

17. A system for adjusting a medical imaging parameter of a medium the system comprises a processing unit configured to:
obtain an optical characteristic of the medium,
adjust the medical imaging parameter as a function of the optical characteristic.

18. The system according to the preceding claim, wherein
the system is a multi-modal system comprising a plurality of different medical imaging modes, wherein
adjusting the medical imaging parameter as a function of the optical characteristic comprises:
selecting and/or confirming a medical imaging mode as a function of the optical characteristic.
